(19) Europäisches Patentamt
European Patent Office
Office européen des brevets

(11) **EP 2 316 333 A1**

(12) **EUROPÄISCHE PATENTANMELDUNG**

(43) Veröffentlichungstag:
**04.05.2011 Patentblatt 2011/18**

(51) Int Cl.:
***A61B 5/024*** *(2006.01)*

(21) Anmeldenummer: **09174346.8**

(22) Anmeldetag: **28.10.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA RS**

(71) Anmelder: **Eidgenössische Materialprüfungs- und Forschungsanstalt EMPA**
**8600 Dübendorf (CH)**

(72) Erfinder:
- **Camenzind, Martin**
  **9500 Wil (CH)**
- **Weder, Markus**
  **9230 Flawil (CH)**
- **Bachmann, Philipp**
  **8700 Küsnacht (CH)**

(74) Vertreter: **Kleinschmidt, Michael**
**Schmauder & Partner AG**
**Patent- und Markenanwälte VSP**
**Zwängiweg 7**
**8038 Zürich (CH)**

(54) **Vorrichtung und Verfahren zum Erkennen und Melden eines Belastungszustandes einer Person**

(57) Um den momentanen Stresszustand einer Person einfach bestimmen zu können wird eine Vorrichtung vorgeschlagen, die die Pulsrate misst und daraus zusätzlich die Herzratenvariabilität - vorzugsweise durch eine schnelle Fouriertransformation - bestimmt. Zusätzlich soll mindestens ein Parameter zur Historie einer der beiden vorgenannten Werte verwendet werden. Vorzugsweise wird die Abweichung der Pulsrate und der Herzratenvariabilität von einer Normgrösse integriert und als zusätzlicher Stressindikator verwendet.

EP 2 316 333 A1

**Beschreibung**

Technisches Gebiet

Die Erfindung betrifft eine Vorrichtung und ein Verfahren zum Erkennen und Melden eines Belastungszustandes einer Person.

Stand der Technik

Für die Ermittlung von Stresszuständen die Pulsrate oder die Herzratenvariabilität zu verwenden, ist seid langem bekannt. Dabei wird der Abstand zwischen zwei Herzschlägen - im Sinne dieser Erfindung - definiert als die Zeit zwischen dem Beginn zweier Kontraktionen der Herzkammern. Dieser Beginn der Kammerkontraktion wird im Elektrokardiogramm (EKG) als R-Zacke dargestellt. Der Abstand zwischen zwei R-Zacken wird üblicherweise als RR-Intervall bezeichnet. Nach einer Mittelung über eine bestimmte Anzahl von RR-Intervallen kann man rechnerisch die Herzfrequenz bestimmen. Die einzelnen Werte der RR-Intervalle schwanken um den so erhaltenen Mittelwert. Dabei können sich die Abweichungen von Schlag zu Schlag ändern. Die Abweichung wird zumeist als Herzratenvariabilität (HRV) bezeichnet. Grundsätzlich kann die Herzfrequenz auch durch eine Druckmessung an einer Arterie vorgenommen werden.

Physiologisch hängt die Herzratenvariabilität (HRV) mit der Fähigkeit des menschlichen Organismus zusammen, die Frequenz des Herzrhythmus anzupassen. Herzratenänderungen, also Veränderungen des zeitlichen Abstandes zwischen zwei Herzschlägen, treten sowohl im Ruhezustand, dann zumeist spontan, wie auch bei bestimmten Veränderungen des Umfeldes, z.B. Belastungen auf. Über physiologische Regulationswege des vegetativen Nervensystems passt ein gesunder Organismus die Herzschlagrate beständig momentanen Erfordernissen an. Körperliche Beanspruchung oder psychische Belastung hat deswegen in der Regel eine Erhöhung der Herzfrequenz zur Folge, die bei Entlastung und Entspannung normalerweise wieder zurückgeht. Dabei zeigt sich eine gute Anpassungsfähigkeit an Belastungen in einer höheren Variabilität der Herzfrequenz. Unter chronischer Stressbelastung ist die Anpassungsfähigkeit reduziert. Insofern bietet - dies ist bekannt - die Herzratenvariabilität für sich genommen schon einen gewissen, allerdings noch sehr unzuverlässigen Indikator für die momentane Stressbelastungen sowie die Stressbelastungsfähigkeit einer Person.

Im Stand der Technik wurden vielfach Verfahren zur Bestimmung des Belastungszustandes (Stresszustandes) einer Person vorgeschlagen, wobei vorgeschlagen wurde, neben der Pulsrate weitere Messgrössen zu verwenden. So wird in der DE 103 19 361 A1 vorgeschlagen, neben der Herzratenvariabilität die Pulswellenlatenz zu verwenden.

In Bezug auf die Auswertung der Herzratenvariabilität wird auf die DE 100 06 154 A1, die DE 10 2006 039 957 A1, sowie die DE 10 2008 030 956 A1 und die EP 1 156 851 B1 verwiesen, in denen der Fachmann verschiedene Verfahren zur Bestimmung finden kann.

Darstellung der Erfindung

Aufgabe der Erfindung ist es, eine Vorrichtung und ein entsprechendes Verfahren zum Erkennen und Melden eines Belastungszustandes einer Person mit einer - gegenüber dem Stand der Technik - erhöhten Zuverlässigkeit zur Verfügung zu stellen.

Die Aufgabe der Erfindung wird - gemäss einem ersten Aspekt - durch ein Verfahren nach Anspruch 1 gelöst. Die Massnahmen der Erfindung haben zunächst einmal zur Folge, dass die mit entsprechenden Gewichtungsfaktoren gewichteten Werte der momentanen Pulsfrequenz und der momentanen Herzratenvariablilität addiert werden. Dies entspricht der bereits aus dem Stand der Technik bekannten Erkenntnis, dass bei einer sehr hohen Pulsfrequenz die Herzratenvariabilität momentan stark abnimmt und damit eine entsprechend verringerte Aussagekraft hat. Andererseits hat im Ruhezustand die Pulsfrequenz eine geringe Aussagekraft über die Belastbarkeit, so dass dann die Herzratenvariabilität wichtiger wird. Dadurch, dass die die genannte Zustandsfunktion mit zumindest einer, vorzugsweise additiven Korrekturgrösse versehen wird, die die Vorgeschichte der Person zumindest innerhalb der letzten 2 Stunden, vorzugsweise innerhalb einer Zeit von ca. 5 Stunden bis max. 72 Stunden, berücksichtigt, wird der Zustandswert des Belastungszustandes - gemäss der vorliegenden Erfindung - erheblich aussagekräftiger.

Gemäss der vorliegenden Erfindung ist es vorteilhaft wenn die Vorgeschichte eine mit einem Gewichtungsfaktor E gewichtete Summe $P_{Hist}$ der gemessenen Pulsfrequenzen P abzüglich einer Funktion $F_1$ zumindest des Ruhepulses $P_0$ berücksichtigt mit

$$P_{Hist} = E * \Sigma (P - G * F_1(P_0))$$

gemäss der Methode eines verschiebenden Fensters ("moving average"). Dabei kann E eine Konstante sein, aber auch eine sich linear mit der Zeit verkleinernde Grösse, die am Anfang der Summierung - vom aktuellen Zeitpunkt aus gesehen den vollen Wert und am Ende der Zeit einen verschwindenden Betrag hat. Alternativ kann die Vorgeschichte auch durch ein Filter, vorzugsweise einen digitalen Tiefpass realisiert werden, der dann die gesamte Vorgeschichte in Bezug auf Stress und Erholung berücksichtigt, schwerpunktmässig aber eine nahe zurückliegende Vorgeschichte.

Dabei ist es sinnvoll, wenn die genannte Funktion $F_1$ weiterhin abhängig ist vom Alter der Person und/oder vom Maximalpuls der Person, vorzugsweise gemessen gemäss dem Conconi-Test.

Besonders vorteilhaft ist es, wenn die Vorgeschichte auch eine mit einem Gewichtungsfaktor H gewichtete Summe einer Funktion $F_2$ der Verhältnisse $LF_{tot}/HF_{tot}$ berücksichtigt mit

$$HRV_{Hist} = H*\Sigma(F_2(LF_{tot}/HF_{tot}))$$

wiederum gemäss der Methode eines verschiebenden Fensters ("moving average"). Dabei kann H wiederum eine Konstante sein, aber auch eine sich linear mit der Zeit verkleinernde Grösse, die am Anfang der Summierung - vom aktuellen Zeitpunkt aus gesehen den vollen Wert und am Ende der Zeit einen verschwindenden Betrag hat. Auch hier kann alternativ die Vorgeschichte auch durch ein Filter, vorzugsweise einen digitalen Tiefpass realisiert werden, der dann die gesamte Vorgeschichte in Bezug auf Stress und Erholung berücksichtigt, schwerpunktmässig aber eine nahe zurückliegende Vorgeschichte.

Besonders einfach und vorteilhaft ist es, wenn die Funktion $F_2(LF_{tot}/HF_{tot})$ eine Normfunktion mit den Werten 1 bei einer momentanen HRV kleiner als einem ersten Grenzwert einer vorbestimmten normierten $HRV_{norm}$, 0 bei einer momentanen HRV grösser als dem ersten Grenzwert der vorbestimmten normierten $HRV_{norm}$, aber kleiner als einem zweiten Grenzwert der vorbestimmten normierten $HRV_{norm}$ und -1 bei einer momentanen HRV grösser als dem zweiten Grenzwert der vorbestimmten normierten $HRV_{norm}$ ist.

Gemäss einem zweiten Aspekt der vorliegenden Erfindung wird die Aufgabe durch eine Vorrichtung gemäss Anspruch 8 gelöst.

Dabei wird durch die Massnahmen der Erfindung gemäss diesem Aspekt eine besonders aussagekräftige Vorrichtung zur Verfügung gestellt, die - verglichen mit entsprechenden Vorrichtungen nach dem Stand der Technik recht einfach und zuverlässig ist, insbesondere, da sie keine überflüssigen Messgrössen ermittelt. Die Vorrichtung umfasst vorteilhafterweise eine Anzeigeeinrichtung, die so eingerichtet ist, dass sie zumindest die Pulsrate, den HRV Wert über eine vorbestimmte oder vorgewählte Zeit sowie die Zustandsfunktion vorzugsweise grafisch darstellen kann.

Gemäss diesem Aspekt der vorliegenden Erfindung ist es vorteilhaft, wenn die Vorrichtung so eingerichtet ist, dass als Vorgeschichte eine mit einem Gewichtungsfaktor E gewichtete Summe $P_{Hist}$ der gemessenen Pulsfrequenzen P abzüglich einer Funktion $F_1$ zumindest des Ruhepulses $P_0$ berücksichtigt wird mit

$$P_{Hist} = E*\Sigma(P-G*F_1(P_0))$$

Dabei kann E eine Konstante sein, aber auch eine sich linear mit der Zeit verkleinernde Grösse, die am Anfang der Summierung - vom aktuellen Zeitpunkt aus gesehen den vollen Wert und am Ende der Zeit einen verschwindenden Betrag hat.

Auch dabei ist es sinnvoll, wenn die genannte Funktion $F_1$ weiterhin abhängig ist vom Alter der Person und/oder vom Maximalpuls der Person, vorzugsweise gemessen gemäss dem Conconi-Test.

Als Funktion F wird in der Vorrichtung alternativ folgende Funktionen vorgeschlagen:

$$F = (c_1 - (Alter - 20) \cdot c_2) \cdot P_0$$

mit typischen Werten von $c_1$ [1.01 ... 6.04], vorzugsweise [1.01 ... 3.00], höchst vorzugsweise 1.2; oder

$$F = P_0 + (P_{max} - P_0) \cdot c_3$$

mit typischen Werten von $c_3$ von [0.10 bis 0.50], vorzugsweise von [0.15 ... 0.30], höchst vorzugsweise 0.20 oder

$$F = P_{mean}$$

wobei $P_{mean}$ ein gemessener, individueller Durchschnittswert des Pulses über einen repräsentativen Tag ist.

Besonders vorteilhaft ist es, wenn die Vorrichtung so eingerichtet ist, dass als die Vorgeschichte auch eine mit einem Gewichtungsfaktor H gewichtete Summe einer Funktion $F_2$ der Verhältnisse $LF_{tot}/HF_{tot}$ berücksichtigt wird mit

$$HRV_{Hist} = H*\Sigma(F_2(LF_{tot}/HF_{tot}))$$

Dabei kann H eine Konstante sein, aber auch eine sich linear mit der Zeit verkleinernde Grösse, die am Anfang der Summierung - vom aktuellen Zeitpunkt aus gesehen den vollen Wert und am Ende der Zeit einen verschwindenden Betrag hat.

Besonders einfach und vorteilhaft ist es, wenn die Funktion $F_2(LF_{tot}/HF_{tot})$ eine Normfunktion mit den Werten 1 bei einer momentanen HRV kleiner als einem ersten Grenzwert einer vorbestimmten normierten $HRV_{norm}$, 0 bei einer momentanen HRV grösser als dem ersten Grenzwert der vorbestimmten normierten $HRV_{norm}$, aber kleiner als einem zweiten Grenzwert der vorbestimmten normierten $HRV_{norm}$ und -1 bei einer momentanen HRV grösser als dem zweiten Grenzwert der vorbestimmten normierten $HRV_{norm}$ ist. Wiederum kann hier alternativ die Vorgeschichte auch durch ein Filter, vorzugsweise einen digitalen Tiefpass realisiert werden, der dann die gesamte Vorgeschichte in Bezug auf Stress und Erholung berücksichtigt, schwerpunktmässig aber eine nahe zurückliegende Vorgeschichte.

Die vorbenannten sowie die beanspruchten und in den nachfolgenden Ausführungsbeispielen beschriebenen, erfindungsgemäss zu verwendenden Elemente unterliegen in ihrer Grösse, Formgestaltung, Materialverwendung und ihrer technischen Konzeption keinen besonderen Ausnahmebedingungen, so dass die in dem jeweiligen Anwendungsgebiet bekannten Auswahlkriterien uneingeschränkt Anwendung finden können. Insbesondere ist das erfinderische Verfahren nicht dazu gedacht, den Gesundheits- oder Krankheitszustand der Person festzustellen.

Wege zur Ausführung der Erfindung

Die erfindungsgemässe Vorrichtung umfasst gemäss einem bevorzugten Ausführungsbeispiel der Erfindung eine Messeinrichtung zur Ermittlung der Pulsrate und der Werte, die zur Berechnung einer Herzratenvariabilität notwendig sind. Dies ist hier ein Pulsmesssensor, kann aber auch alternativ ein elektrischer Sensor zur Messung von elektrischen kardiografischen Messwerten sein, sowie ein Anzeigegerät. Weiterhin umfasst die Vorrichtung eine Schnittstelle zur Eingabe von personenbezogenen Grössen, die insbesondere zur Ermittlung der erfindungsgemäss zu verwendenden Historie notwendig sind. Kernstück der Vorrichtung ist eine Recheneinrichtung, die die notwendige Messdatenerfassung kontrolliert, die Messdatenaufbereitung in der notwendigen digitalen Form durchführt, die Datenverarbeitung durchführt und die Anzeige bedient.

Die Benutzung der vorgeschlagenen Vorrichtung geschieht grundsätzlich nach folgendem Schema:

Zunächst wird eine Parametrisierung durchgeführt, was selbstverständlich pro Person nur einmal notwendig ist. Die Parametrisierung umfasst Angaben zu Alter, Geschlecht, und allenfalls sonstige Korrekturfaktoren, sofern diese zur Feinabstimmung verwendet werden sollen.

Weiterhin ist ein Schritt durchzuführen, der im Folgenden als Kalibrierung bezeichnet wird und zyklisch, z.B. jährlich anlässlich Tauglichkeitstests durchzuführen ist. Dabei werden z.B. der Ruhepuls $p_0$ - z.B. ausgeruht am morgen vor Frühstück oder entspannt nach 5' liegen - sowie - falls diese Option aktiviert ist - der Maximalpuls $p_{max}$ - z.B. mittels eines Conconi-Tests oder eines ähnlichen Belastungstests ermittelt. Weiterhin wird ein normierter Wert zur Herzratenvariabilität $HRV_{norm}$ als Basiswert in Ruhe ermittelt. Falls auch z.B. die anaerobe Schwelle in die Funktion eingehen soll, kann auch diese bestimmt und eingegeben werden.

Es ist vorgesehen, dass die Vorrichtung bei allen Einsätzen getragen werden sollte. Dadurch ergibt sich ein Betrieb mit einem Einschalten ca. von mindestens 2 min vor dem Einsatz, wobei es zur vollständigen Ermittlung der Historie selbstverständlich besser ist, das Einschalten mehrere Stunden vor dem Einsatz durchzuführen. Auch ein Ausschalten sollte - wenn möglich - nicht sofort nach Einsatz erfolgen, damit Erholungsdaten ermittelt werden können (Erholungskurve).

Die Auswertung erfolgt durch die Recheneinrichtung der Vorrichtung. Die Anzeige umfasst im angegebenen Ausführungsbeispiel eine Darstellung der kontinuierlich erfassten Datenerfassung, z.B. mit einer zeitlichen Darstellung von 10 Werten pro Sekunde für den Wert HRV, eine gemittelte Datenanzeige, abhängig von Anwendung, sowie eine Auswertung des Wertes HRV mittels einer schnellen Fouriertransformation FFT mit einem Fenster von ca. 2 Minuten.

Der Berechnung einer Zustandsgrösse (Stressgrösse) ist im vorliegenden Ausführungsbeispiel eine Funktion der Pulsrate P und der Herzratenvariabilität HRV, sowie der Vorgeschichte beider Grössen ($p_{hist}$, $HRV_{hist}$). Zudem ist es im vorliegenden Ausführungsbeispiel vorgesehen, Anpassungen an die Daten der Person vorzunehmen, die bei zyklischen Kalibrierungen erfolgen. Die Vorgeschichte dient zur Berücksichtigung vorheriger Einsätze bzw. soll miteinbeziehen, wenn die Person vorab schon Stress ausgesetzt war (Reduktion der Leistungsfähigkeit, bzw. Einsatzdauer). Die verwendete Zustandsgrösse errechnet sich

$$Stress = A \cdot P + B \cdot P_{hist} + C \cdot HRV + D \cdot HRV_{hist}$$

P: aktueller Puls
HRV: aktueller Wert Herzratenvariabilität
$P_{hist}$: Vorgeschichte Puls
$HRV_{hist}$: Vorgeschichte HRV

Dabei wird als

$$P_{hist} = \sum_{-72h}^{-2\min} E(t) \cdot (p - G \cdot F(P_0, P_{max}, Alter, ..))$$

verwendet mit
$P_o$: Ruhepuls, gemessen z.B. nach Aufstehen, vor Frühstück
$P_{max}$: Maximalpuls, ermittelt z.B. mittels des Conconi-Tests
Als einfache Funktion F kann in der Vorrichtung zwischen verschiedenen Funktionen gewählt werden, nämlich

$$F = (c_1 - (Alter - 20) \cdot c_2) \cdot P_0$$

mit typischen Werten von $c_1$ [1.01 ... 6.04], vorzugsweise [1.01 ... 3.00], höchst vorzugsweise 1.2;
oder

$$F = P_0 + (P_{max} - P_0) \cdot c_3$$

mit typischen Werten von $c_3$ von [0.10 bis 0.50], vorzugsweise von [0.15 ... 0.30], höchst vorzugsweise 0.20
oder

$$F = P_{mean}$$

mean

wobei P$_{mean}$ ein gemessener, individueller Durchschnittswert des Pulses über einen repräsentativen Tag ist.

Es können aber auch andere Funktionen vorgegeben werden, die weitere historische und personenbezogene Werte mit verwenden, z.B. das Geschlecht, Körpergrössen wie den Body-Mass-Index etc.

Der Abstand zwischen zwei Herzschlägen wird meistens definiert als die Zeit zwischen dem Beginn zweier Kontraktionen der Herzkammern *(R-Zacke)*. Der Abstand zwischen zwei R-Zacken wird daher als *RR-Intervall* bezeichnet. Die realen RR-Intervalle schwanken um die mittlere Herzfrequenz, wobei diese Abweichungen sich sogar von Schlag zu Schlag ändern können; dies bezeichnet man als Herzfrequenz- oder Herzratenvariabilität (HRV).

Es gibt verschiedene Kennwerte, die sich für die Auswertung im Stresssensor eignen, wobei im vorliegenden Ausführungsbeispiel der Quotient der Integrale verschiedener Frequenzbereiche nach einer Fouriertransformation verwendet wird

HF (0.18-0.40 Hz)
LF (0.04-0.15 Hz)

$$\text{HRV} = \frac{LF_{tot}}{HF_{tot}} = \frac{\int_{0.04}^{0.15} FFT(p)}{\int_{0.18}^{0.4} FFT(p)}$$

Dabei wird der Wert HF zumeist einer parasympathischen Beeinflussung, der Wert LF einer sympathischen Beeinflussung zugeordnet.

Alternativ kann auch eine statistische Zeitanalyse, nämlich die Streuung zwischen den RR- Abständen (z.B. RMSSD oder pNN50) verwendet werden. Die Vorrichtung ist so eingerichtet, dass sie diese Option wählen kann.

In beiden Fällen sind tiefere Werte schlechter als höhere Werte, da grössere Streuungen auf ein gesundes Herz und das normale Wechselspiel zwischen Sympathikus und Parasympathikus hindeuten.

Die Fouriertransformation erfolgt in der Vorrichtung gemäss diesem Ausführungsbeispiel anhand der RR-Daten über ein Intervall von ca. 2 Minuten. Dies kann aber abhängig von der Anwendung angepasst werden mit dem Ziel, keine zu grosse Latenz, aber genügend Punkte für einen aussagekräftigen Wert zu erhalten. Die Berechnung des HRV geschieht nach bekannten Algorithmen für eine Schnelle Fourier-Transformation).

Die Historienberechnung der Herzratenvariabilität wird im vorliegenden Ausführungsbeispiel wie folgt vorgenommen: Der Normalwert für die Herzratenvariabilität HRV$_{norm}$ wird für die Person bei einer Kalibrierung erhoben. Im Allgemeinen liegt der Wert zwischen 1.5 und 2.0.

$$\text{HRV}_{hist} = H \cdot \sum_{-72h}^{-2\min} F(HRV) \quad ; \quad HRV = \frac{LF_{tot}}{HF_{tot}}$$

Bsp. für Funktion F

$$1: \quad HRV < d_1 \cdot HRV_{norm}$$

$$F = \quad 0: \quad d_1 \cdot HRV_{norm} < HRV < d_2 \cdot HRV_{norm}$$

$$-1: \quad HRV > d_2 \cdot HRV_{norm}$$

wobei d$_1$ <= d$_2$ ist und d$_1$ einen Bereich von [0.2 ... 0.6], vorzugsweise [0.3 ... 0.5]und höchst vorzugsweise von 0.4 und d$_2$ einen Bereich von [0.5 ... 0.9], vorzugsweise [0.55 ... 0.75]und höchst vorzugsweise von 0.65 hat.

Nachstehend werden Beispiele anhand von Probanden angegeben.

|   | Alter | $p_0$ | $p_{max}$ | $HRV_{norm}$ |
|---|---|---|---|---|
| A | 20 | 60 | 195 | 2.5 |
| B | 50 | 75 | 170 | 2 |

|  | Proband A | | | | Proband B | | | |
|---|---|---|---|---|---|---|---|---|
| p | $P_{hist}$ V1 | $P_{hist}$ V2 | HRV1) | $HRV_{hist}$ | $P_{hist}$ V1 | $p_{hist}$ V2 | HRV1) | $HRV_{hist}$ |
| 60 | -12.0 | -20.3 | 2.5 | -1 | -7.5 | -29.3 | 2.00 | -1 |
| 105 | 33.0 | 24.8 | 2.0 | -1 | 37.5 | 15.8 | 1.25 | 0 |
| 130 | 58.0 | 49.8 | 1.5 | 0 | 62.5 | 40.8 | 0.50 | 1 |
| 165 | 93.0 | 84.8 | 0.5 | 1 | 97.5 | 75.8 | 0.15 | 1 |

V1) $F = (1.2-(Alter-20)\cdot 0.01)\cdot P_0$
V2) $F = P_0 + (P_{max} - P_0)\cdot 0.15$

## Patentansprüche

1. Verfahren zum Erkennen und Melden eines Belastungszustandes einer Person, wobei das Verfahren folgende Schritte umfasst:

   • fortlaufendes Erfassen der Daten zumindest einer körperphysiologischen Führungsgrösse, wobei die zumindest eine körperphysiologische Führungsgrösse zumindest die Pulsfunktion der Person umfasst,
   • fortlaufendes Verarbeiten der Daten der zumindest einen körperphysiologischen Führungsgrösse zu einer momentanen Zustandsfunktion der Person,
   • Vergleichen der gewonnenen momentanen Zustandsfunktion der Person mit einem Alarmkriterium, wobei
   • aus der körperphysiologischen Führungsgrösse fortlaufend zumindest eine momentane Pulsfrequenz P und eine momentane Herzratenvariabilität HRV gebildet wird,
   **dadurch gekennzeichnet, dass**
   • die Zustandsfunktion eine Funktion der momentanen Pulsfrequenz und der momentanen Herzratenvariabilität ist, vorzugsweise eine Linearkombination der momentanen Pulsfrequenz P und der momentanen Herzratenvariabilität

$$Z = A * P + B * HRV$$

   mit vorzugsweise festen Gewichtungsfaktoren A und B, wobei die Herzratenvariabilität vorzugsweise bestimmt wird durch den Quotienten der Integrale verschiedener Frequenzbereiche nach einer Frequenzanalyse, vorzugsweise einer Fouriertransformation, $LF_{tot}/HF_{tot}$, wobei die genannte Zustandsfunktion mit zumindest einer, vorzugsweise additiven Korrekturgrösse versehen wird, die die Vorgeschichte der Person zumindest innerhalb der letzten 0,5 Stunden berücksichtigt.

2. Verfahren nach Anspruch 1, **dadurch gekennzeichnet, dass** die Vorgeschichte eine mit einem Gewichtungsfaktor E gewichtete Summe $P_{Hist}$ der gemessenen Pulsfrequenzen P abzüglich einer Funktion $F_1$ zumindest des Ruhepulses Po berücksichtigt mit

$$P_{Hist} = \Sigma E(t)(P-G*F_1(P_0))$$

3. Verfahren nach Anspruch 2, **dadurch gekennzeichnet, dass** die genannte Funktion $F_1$ weiterhin abhängig ist vom Alter der Person und/oder vom Maximalpuls der Person, vorzugsweise gemessen gemäss dem Conconi-Test.

4. Verfahren nach Anspruch 1, **dadurch gekennzeichnet, dass** der Wert $P_{Hist}$ mittels eines Tiefpassfilter unter Berücksichtigung der zuvor gemessenen Pulsmesswerte ermittelt wird.

5. Verfahren nach einem der Ansprüche 1 bis 4, **dadurch gekennzeichnet, dass** die Vorgeschichte eine mit einem Gewichtungsfaktor H gewichtete Summe einer Funktion F2 der Verhältnisse $LF_{tot}/HF_{tot}$ berücksichtigt wird mit

$$HRV_{Hist} = \Sigma H(t)F_2(LF_{tot}/HF_{tot})$$

6. Verfahren nach einem der Ansprüche 1 bis 4, **dadurch gekennzeichnet, dass** die Vorgeschichte weiterhin einen mit einem Gewichtungsfaktor H gewichteten Wert $HRV_{Hist}$ aufweist, der mittels eines Tiefpassfilter unter Berücksichtigung der zuvor gemessenen HRV-Werte ermittelt wird.

7. Verfahren nach Anspruch 5 oder 6, **dadurch gekennzeichnet, dass** die Funktion $F_2(LF_{tot}/HF_{tot})$ eine Normfunktion mit den Werten 1 bei einer momentanen HRV kleiner als einem ersten Grenzwert einer vorbestimmten normierten $HRV_{norm}$ 0 bei einer momentanen HRV grösser als dem ersten Grenzwert der vorbestimmten normierten $HRV_{norm}$, aber kleiner als einem zweiten Grenzwert der vorbestimmten normierten $HRV_{norm}$ und -1 bei einer momentanen HRV grösser als dem zweiten Grenzwert der vorbestimmten normierten $HRV_{norm}$ ist.

8. Vorrichtung zum Erkennen und Melden eines Belastungszustandes einer Person, mit:

   • einer Erfassungseinrichtung zum fortlaufenden Erfassen der Daten zumindest einer körperphysiologischen Führungsgrösse, wobei die zumindest eine körperphysiologische Führungsgrösse zumindest die Pulsfunktion der Person umfasst,
   • einer Verarbeitungseinrichtung zum fortlaufenden Verarbeiten der Daten der zumindest einen körperphysiologischen Führungsgrösse zu einer momentanen Zustandsfunktion der Person,
   • eine Vergleichseinrichtung zum Vergleichen der gewonnenen momentanen Zustandsfunktion der Person mit einem Alarmkriterium, wobei
   • die Verarbeitungseinrichtung so ausgebildet ist, dass aus der körperphysiologischen Führungsgrösse fortlaufend zumindest eine momentane Pulsfrequenz P und eine momentane Herzratenvariabilität HRV gebildet werden kann,
   **dadurch gekennzeichnet, dass**
   • die Zustandsfunktion eine Funktion der momentanen Pulsfrequenz und der momentanen Herzratenvariabilität ist, vorzugsweise eine Linearkombination der momentanen Pulsfrequenz und der momentanen Herzratenvariabilität

$$Z = A * P + B * HRV$$

   mit vorzugsweise festen Gewichtungsfaktoren A und B, wobei die Herzratenvariabilität vorzugsweise bestimmt wird durch den Quotienten der Integrale verschiedener Frequenzbereiche nach einer Frequenzanalyse, vorzugsweise einer Fouriertransformation, $LF_{tot}/HF_{tot}$,
   und die Verarbeitungseinrichtung so eingerichtet ist, dass die genannte Zustandsfunktion mit zumindest einer, vorzugsweise additiven Korrekturgrösse versehen wird, die die Vorgeschichte der Person zumindest innerhalb der letzten 0,5 Stunden berücksichtigt.

9. Vorrichtung nach Anspruch 8, weiterhin **gekennzeichnet durch** eine Anzeigeeinrichtung, wobei die Anzeigeeinrichtung so eingerichtet ist, dass sie zumindest die Pulsrate, den HRV Wert über eine vorbestimmte oder vorgewählte Zeit sowie die Zustandsfunktion vorzugsweise grafisch darstellen kann.

**10.** Vorrichtung nach Anspruch 8 oder 9, **dadurch gekennzeichnet, dass** sie so eingerichtet ist, dass als Vorgeschichte eine mit einem Gewichtungsfaktor E gewichtete Summe $P_{Hist}$ der gemessenen Pulsfrequenzen P abzüglich einer Funktion $F_1$ zumindest des Ruhepulses $P_0$ berücksichtigt wird mit

$$P_{Hist} = \Sigma E(t)(P-G*F_1(P_0))$$

oder dass der Wert $P_{Hist}$ mittels eines Tiefpassfilter unter Berücksichtigung der zuvor gemessenen Pulsmesswerte ermittelt wird.

**11.** Vorrichtung nach Anspruch 10, **dadurch gekennzeichnet, dass** die genannte Funktion $F_1$ weiterhin abhängig ist vom Alter der Person und/oder vom Maximalpuls der Person, vorzugsweise gemessen gemäss dem Conconi-Test.

**12.** Vorrichtung nach Anspruch 10 oder 11 **dadurch gekennzeichnet, dass** sie die Funktion

$$F = (c_1 - (Alter - 20) \cdot c_2) \cdot P_0$$

Werten von $c_1$ [1.01 ... 6.04], vorzugsweise [1.01 ... 3.00],
höchst vorzugsweise 1.2;
umfasst.

**13.** Vorrichtung nach Anspruch 10 oder 11 **dadurch gekennzeichnet, dass** sie die Funktion

$$F = P_0 + (P_{max} - P_0) \cdot c_3$$

Werten von $c_3$ von [0.10 bis 0.50], vorzugsweise von [0.15 ... 0.30], höchst vorzugsweise 0.20
umfasst.

**14.** Vorrichtung nach Anspruch 10 oder 11, **dadurch gekennzeichnet, dass**
$F = P_{mean}$ ist, wobei $P_{mean}$ ein gemessener, individueller Durchschnittswert des Pulses über einen repräsentativen Tag ist.

**15.** Vorrichtung nach einem der Ansprüche 8 bis 13, **dadurch gekennzeichnet, dass** sie so eingerichtet ist, dass die Vorgeschichte eine mit einem Gewichtungsfaktor H gewichtete Summe einer Funktion $F_2$ der Verhältnisse $LF_{tot}/HF_{tot}$ berücksichtigt mit

$$HRV_{Hist} = \Sigma H(t)F_2(LF_{tot}/HF_{tot})$$

oder mit einem Wert $HRV_{Hist}$, der mittels eines Tiefpassfilter unter Berücksichtigung der zuvor gemessenen HRV-Werte ermittelt wird, wobei die Funktion $F_2(LF_{tot}/HF_{tot})$ vorzugsweise eine Normfunktion mit den Werten 1 bei einer momentanen HRV kleiner als einem ersten Grenzwert einer vorbestimmten normierten $HRV_{norm}$, 0 bei einer momentanen HRV grösser als dem ersten Grenzwert der vorbestimmten normierten $HRV_{norm}$, aber kleiner als einem zweiten Grenzwert der vorbestimmten normierten $HRV_{norm}$ und -1 bei einer momentanen HRV grösser als dem zweiten Grenzwert der vorbestimmten normierten $HRV_{norm}$ ist.

Europäisches
Patentamt

European
Patent Office

Office européen
des brevets

# EUROPÄISCHER RECHERCHENBERICHT

Nummer der Anmeldung

EP 09 17 4346

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2007/219059 A1 (SCHWARTZ MARK H [US] ET AL) 20. September 2007 (2007-09-20) * Absätze [0039], [0040], [0049], [0095], [0 96], [0111], [0168], [0169] * ----- | 1-15 | INV. A61B5/024 |
| X | WO 2007/057032 A1 (ENERGY LAB TECHNOLOGIES GMBH [DE]; SCHWARZ GEROLD [DE]; WEITL MARC [DE] 24. Mai 2007 (2007-05-24) * Seite 9, Zeile 11 - Seite 13, Zeile 30 * ----- | 1-15 | |
| X | BOZHOKIN ET AL: "Analysis of the heart rate variability using stress tests" HUMAN PHYSIOLOGY, Bd. 34, Nr. 4, 9. August 2008 (2008-08-09) , Seiten 461-467, XP002575014 ISSN: 0362-1197 * das ganze Dokument * ----- | 1-15 | |
| | | | RECHERCHIERTE SACHGEBIETE (IPC) A61B |

Der vorliegende Recherchenbericht wurde für alle Patentansprüche erstellt

| Recherchenort | Abschlußdatum der Recherche | Prüfer |
|---|---|---|
| Den Haag | 25. März 2010 | Bengtsson, Johan |

**ANHANG ZUM EUROPÄISCHEN RECHERCHENBERICHT**
**ÜBER DIE EUROPÄISCHE PATENTANMELDUNG NR.**

EP 09 17 4346

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-03-2010

| Im Recherchenbericht angeführtes Patentdokument | Datum der Veröffentlichung | Mitglied(er) der Patentfamilie | Datum der Veröffentlichung |
|---|---|---|---|
| US 2007219059 A1 | 20-09-2007 | KEINE | |
| WO 2007057032 A1 | 24-05-2007 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10319361 A1
- DE 10006154 A1
- DE 102006039957 A1
- DE 102008030956 A1
- EP 1156851 B1